# EUROPEAN PATENT SPECIFICATION

(11) **EP 3 705 104 B1**
(45) Date of publication and mention of the grant of the patent: **07.06.2023**
(21) Application number: 17930482.9
(22) Date of filing: 08.11.2017
(51) Int. Cl.: A61H 3/00, A61F 5/01, A61H 1/02

(54) **ANKLE BRACE OR ANKLE EXOSKELETON**
SPRUNGGELENKORTHESE ODER SPRUNGGELENK-EXOSKELETT
ARTICULATION TIBIO-TARSIENNE D'UNE ORTHÈSE OU D'UN EXOSQUELETTE

(30) Priority: 31.10.2017 RU 2017137860
(43) Date of publication of application: 09.09.2020
(73) Proprietor: Obshchestvo S Ogranichennoy Otvetstvennost'yu "Exoatlet", Moscow 119121 (RU)
(72) Inventor: PISMENNAYA, Elena Valentinovna, Moscow 119192 (RU); TOLSTOV, Kirill Mihailovich, Moscow 129347 (RU)
(74) Representative: Jeck, Anton
(86) International application number: PCT/RU2017/000839
(87) International publication number: WO 2019/088869

(56) References cited:
- WO-A1-2016/067229
- FR-A3- 3 004 105
- FR-A3- 3 004 105
- JP-A- 2012 099 790
- US-A- 2 567 195
- US-A- 3 805 773
- US-A1- 2015 342 820
- US-A1- 2015 342 820
- US-B2- 9 492 302
- US-B2- 9 662 262

## Description

The invention relates to the field of human necessities and can be used in medicine, in particular, in the treatment of patients with loss or injury of locomotor function of the lower extremities. The main purpose of the invention is to restore/form compensatory walking and/or to improve gait in persons with impaired, lost or unformed ability to control movements of the lower extremities and, besides, to improve the physical condition and quality of life of individual groups of patients. Document FR 3 004 105 A3 represents the closest prior art.

The invention can be used in the rehabilitation of persons who have lost their locomotor capabilities due to injuries and diseases of the spine, traumatic brain injury, stroke and other diseases leading to central paralysis, as well as due to defects or disorders of the central nervous system, including infant cerebral palsy.

The ankle link of exoskeleton described in the published international application WO 2013/049658, A61H3/00, 2013 is known to contain two struts located on both sides of the human carrier's ankle. At the lower ends of the struts, a C-shaped bracket is hinged, which is pivotally connected to the foot support platform in the heel part.

The ankle link of a passive type exoskeleton is described in patent RU 2362598, A62B99/00, A61H3/00, 2007. It includes a support lever made in the form of a spatial C-shaped shell, which can be fixed on the front of the ankle with fastening elements. On the outer surface of the shell, horizontal slots are made for moving of the limb or foot around a common vertical axis.

The support lever is linked to the connector assembly of the foot support via a slider that provides rotation around a common vertical axis. The connector assembly of the foot represents a fork, which is connected to the support foot via hinge. The support foot is inserted into footwear via hinges located on both sides of the foot. It provides three degrees of freedom of the foot moving and the possibility of angular displacement around the axes XYZ.

The ankle link of a passive type exoskeleton is described in patent RU 2563209, A61H3/00, 2014. It contains a supporting element connected to the ankle fork with a screw through a sliding bearing. The ankle fork has the form of rocker arms with lowered parallel ends. The ankle fork has lateral tides limiting the lateral amplitude (right-left) of the foot's oscillation, in order to avoid ligament damage.

Retainer-latches are attached to the ends of the ankle fork via sliding bearings, providing a detachable connection of the support platform. The support platform bracket is inserted into the shoe, for example, under the insole. On the outside of the shoe, the side rests of the support platform linked with the retainer-latches are applied from 2 sides, for example, in the form of a trapezoid plate with slots in the upper part, and are screwed to the support bracket. This device is accepted as a prototype.

Disadvantage of known devices is the complexity of the design, high weight, low load capacity. Besides, they do not allow movement around the axes passing through the center of the ankle joint of the exoskeleton operator. It negatively affects the ergonomic characteristics of the human-machine system operator-exoskeleton.

The technical result of the proposed invention is to expand the variety of technical means of ankle links of orthosis and exoskeletons by creating a universal link suitable for carriers with different degrees of mobility of the ankle joint.

In addition, increasing the stability and safety of the human operator is also the technical result.

The specified technical result is achieved due to the fact that the ankle link or ankle exoskeleton contains the base (carrying, supporting) element, the first lever, the first strut, the second strut and the foot support.

The first lever is mounted on the base element and can be rotated. The first strut is hinged at the upper end to the first lever, and the second strut is hinged at the upper end to the base element. The foot support is connected by the first lateral side to the lower end of the first strut and by the second lateral side to the lower end of the second strut via hinges having at least two degrees of freedom.

In a specific case of execution, the base element can be made with the possibility of connecting to the ankle of the human carrier.

In a specific case of execution, the ankle link can be equipped with connecting elements, installed on the base element. Connecting elements are located on the lateral sides of the human carrier's ankle and designed for connection to the knee joint of the orthosis or exoskeleton.

In a particular case of execution, the connecting elements can be made with the ability to adjust their position in the horizontal plane relative to the base element.

It is preferable to construct an ankle link in such a way that the first and second struts can be able to change their length.

In a particular case of execution, the first lever can be made with the possibility of limiting its rotation or with the possibility of locking in a specified position.

It is preferable to construct an ankle link in such a way that the first lever can be able to change its length.

In a particular case of execution, the end of the first lever can be rotated and located in a plane parallel to the sagittal plane.

In a preferred embodiment, the hinge connection of the first strut with a first lever is formed by the pin, located at the end of the first lever, and a bearing, located on the first strut. The hinge connection of the second strut with the base element is formed by the pin, located on the base element in a plane parallel to the sagittal plane, and the bearing, located on the second strut.

In the preferred embodiment, each hinge connecting the corresponding strut to the foot support is made in the form of a spherical hinge.

In a particular case of execution, the first strut can be made of two parts that can be rotated relative to each other and then fixed. Wherein the first part of the first strut is connected to the first lever and the second part is connected to the spherical hinge.

In a specific case of execution, the ankle link additionally contains a mechanical and/or pneumatic springy element, pivotally connected at one end to the base element, and the other end to the heel part of the foot support.

In a specific case of execution, the ankle link additionally contains a linear displacement drive, pivotally connected at one end to the base element, and at the other end to the heel part of the foot support.

In a specific case of execution, the ankle link additionally contains a bracket connected to the heel and lateral sides of the foot support and rigidly fixed to the first and second struts.

The above is a summary of the invention and thus may contain simplifications, generalizations, inclusions and/or exclusions of details; therefore, specialists in this field of technology should take into account that this summary of the invention is only illustrative one and does not imply any limitation.

For a better understanding of the proposed technical solutions the following is a description of a specific implementation, which isn't the limiting example of practical realization of the ankle link in accordance with the claimed invention, with reference to the drawings, in which are presented the following.
Fig.1 shows the ankle link of the right leg, front view, axonometry (elements of attachment to the ankle and shoes of the wearer are not shown).
Fig.2 shows the ankle link of the right leg, rear view, axonometry (elements of attachment to the ankle and shoes of the wearer are not shown).
Fig.3 shows the ankle link of the right leg, lateral side view.
Fig.4 shows the ankle link of the right leg, right view.
Fig.5 shows the ankle link of the right leg, left view.
Fig.6 shows the ankle link of the right leg, rear view.
Fig.7 shows the ankle link of the right leg, front view.
Fig.8 shows the view A in Fig.6.
Fig.9 shows the ankle link of the right leg with the abducted foot support, top view.
Fig.10 shows the view B in Fig.6, the parts of the first strut are rotated relative to each other.
Fig.11 shows the view A in Fig.6, the parts of the first strut are rotated relative to each other.
Fig.12 shows the ankle link of the right leg with the abducted foot support, front view, axonometry.
Fig.13 shows the ankle link of the right leg with a springy element, front view, axonometry.
Fig.14 shows the ankle link of the right leg with a springy element, rear view, axonometry.
Fig.15 shows the ankle link of the right leg with a fixing bracket, front view, axonometry.
Fig.16 shows the ankle link of the right leg with a fixing bracket, rear view, axonometry.
Fig.17 shows the ankle link of the right leg with the disconnected foot support, left view, axonometry.

It should be noted that the drawings show only those details that are necessary for understanding the substance of the proposal, and the accompanying equipment, which is well known to specialists in this field, is not shown here.

As noted earlier, the main purpose of the claimed ankle link is its use in ankle link of orthosis or exoskeletons of the lower limbs, both active and passive, to restore or improve gait in persons with impaired, lost or unformed ability to control the movements of the lower limbs due to various diseases and injuries. At the same time, depending on the degree of damage, each patient requires a specific adjustment of the mobility of the ankle, up to its complete fixation.

It is obvious that the claimed ankle link can also be used in passive exoskeletons for healthy people when performing work related to the transportation of various loads. In this case, the degree of mobility of the ankle link is determined by the mobility of the human ankle joint.

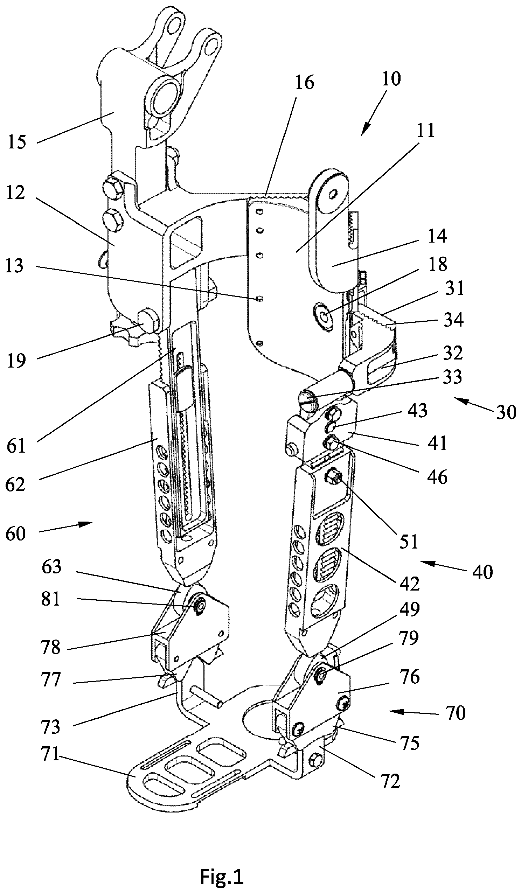

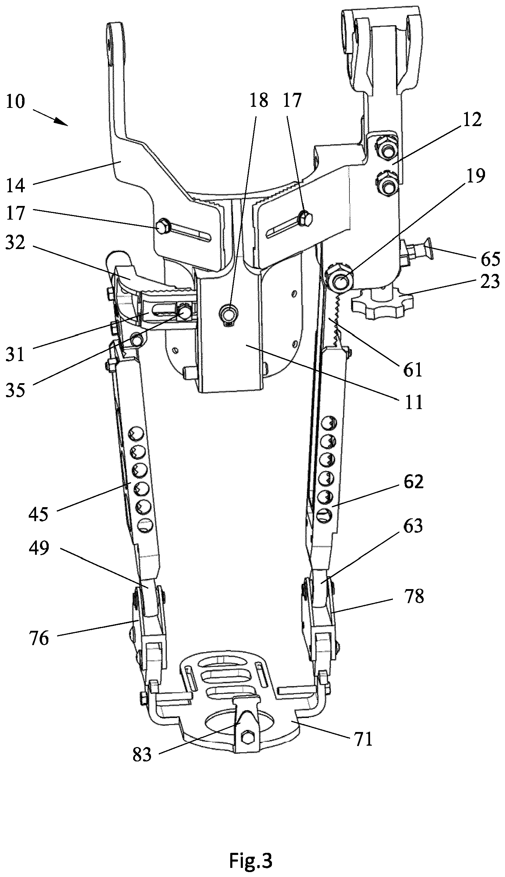

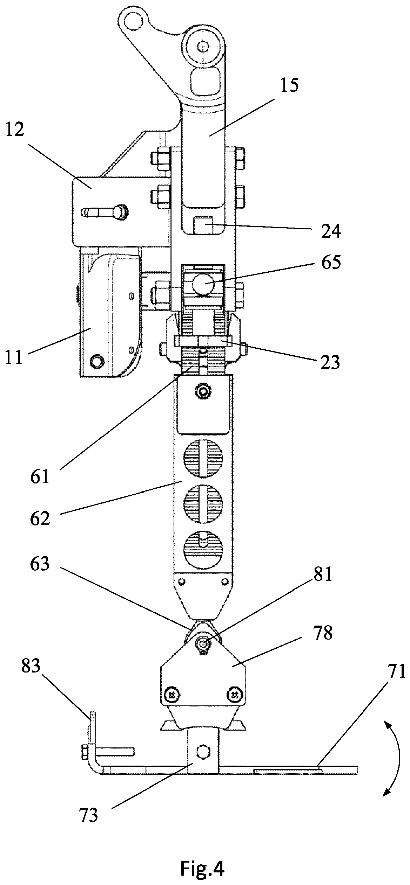

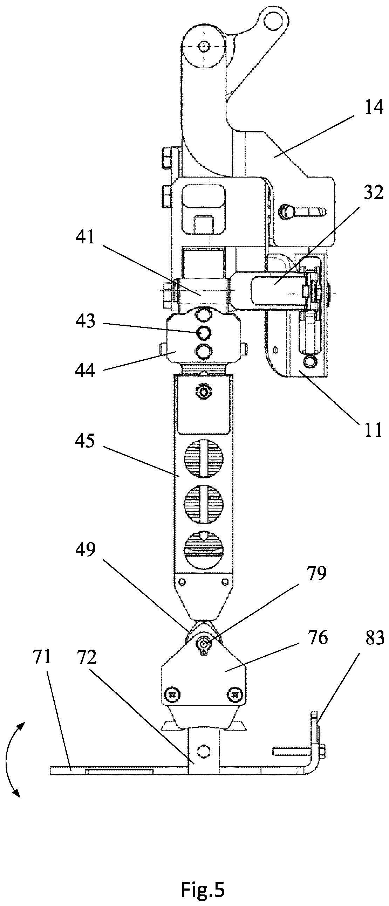

As shown in Fig. 1 - 5, the ankle link for the orthosis of lower limbs or the exoskeleton contains the base element 10, which, in this particular example, is designed for connecting to the human carrier's ankle. In general, the connection of the ankle link with the human carrier's ankle is not necessary. It is enough to mount it on the hip and foot, as is the case for some designs of passive exoskeletons.

The base element 10 consists of a support element 11 and a bracket 12. On the support element 11 of the base element 10, there are holes 13 to which fixing belts or other similar means are attached (not shown in Fig.). Means are designed to fix the ankle link on the ankle of the human carrier.

Connecting element 14 is installed on the support element 11, located on one side of the ankle of the human carrier, for connection to the knee joint of the orthosis or exoskeleton. Connecting element 15 is installed on the bracket 12, located on the other side of the ankle of the human carrier. In this example, the connecting element 15 is made in the form of a lug designed for connection to the drive of the ankle link of the active exoskeleton.

To fit the ankle link to a certain person-carrier, the bracket 12 and the connecting element 14 are mounted on the support element 11 with the possibility of adjusting their position relative to the support element 11 in the horizontal plane. To do this, the bracket 12 and the connecting element 14 are able to move relative to the support element 11 at an angle to the sagittal plane with subsequent fixation.

Fixing the bracket 12 and the connecting element 14 on the support element 11 may be carried out by any method known in the art. In this particular example, fixation is carried out by means of teeth 16 made on the support element 11, on the bracket 12 and the connecting element 14, as well as screws 17.

The pin 18 is on the support element 11, and the pin 19 is on the bracket 12. The first lever 30 is installed on the pin 18.The first lever 30 contains the base 31 and the bracket 32. The end of the bracket 32 has a curved shape, turned towards the lower leg of the human carrier, and lies in a plane parallel to the sagittal. The pin 33 lying in a plane parallel to the sagittal is at the end of the bracket 32.

To fit the ankle link to a certain person-carrier, the length of the lever 30 may vary. To do this, the bracket 32 is installed on the base 31, with the possibility of moving and then fixing. Fixing the bracket 32 on the base 31 may be carried out by any method known in the art. In this particular example, fixation is carried out by means of teeth 34, made on the base 31 and the bracket 32, as well as a screw 35.

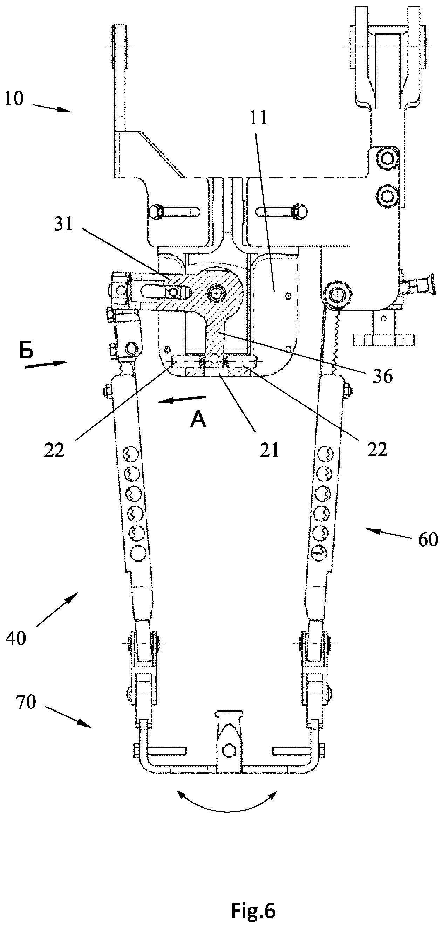

As shown in Fig.6, the base 31 of the lever 30 has a stop 36 placed in the hole 21 of the support element 11 and designed to limit the angle of rotation of the lever 30 or, in other words, the mobility of the ankle link. To adjust the specific angle of rotation required for the patient, the adjustment screws 22 mounted on the support element 11 are used. In addition, if necessary, the lever 30 can be fully fixed with screws 22.

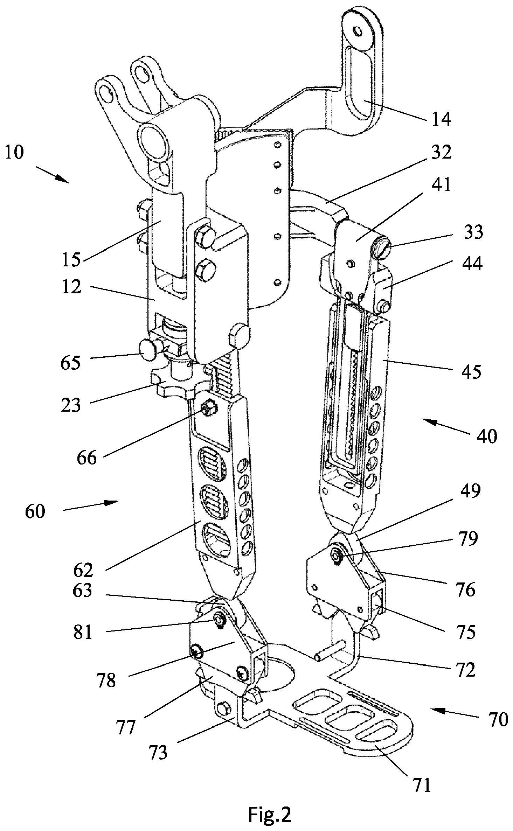

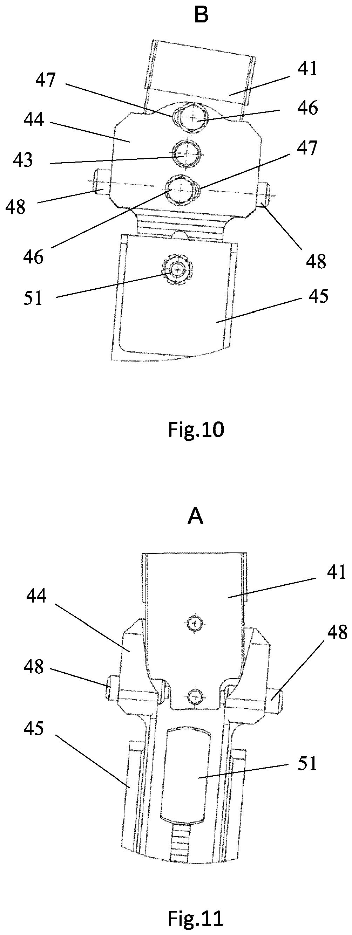

The first strut 40 contains the first 41 and second 42 parts. The first part 41 is mounted on the pin 33 of the lever 30 and has a pin 43. The second part 42 is mounted on the pin 43 with the possibility of rotation and further fixation and consists of the first 44 and the second 45 elements (Fig.2). Fixing the second part 42 relative to the first part 41 of the first strut 40 is carried out by screws 46 located in the circular holes 47 of the first element 44 of the second part 42 of the first strut 40 (Fig. 10).

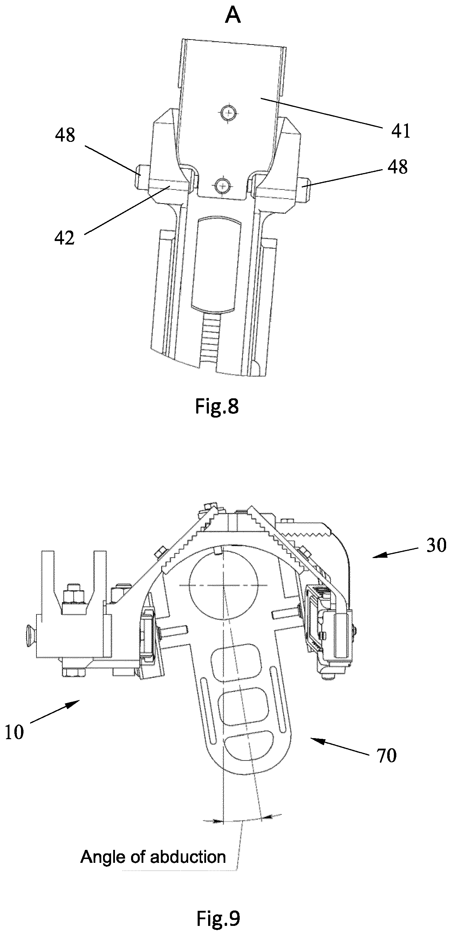

The angle of rotation of the first 41 and second 42 parts is regulated by setting screws 48 (Fig. 8, 10, 11). At the lower end of the second element 45 there is a spherical hinge in the form of a standard spherical bearing 49.

Such implementation of the first strut 40 allows you to realize two functions, namely: the function of adjusting the angle of abduction/adduction of the foot and the function of general adjustment of the ankle link for a certain person by changing the position of the bracket 12 and the connecting element 14 relative to the support element 11.

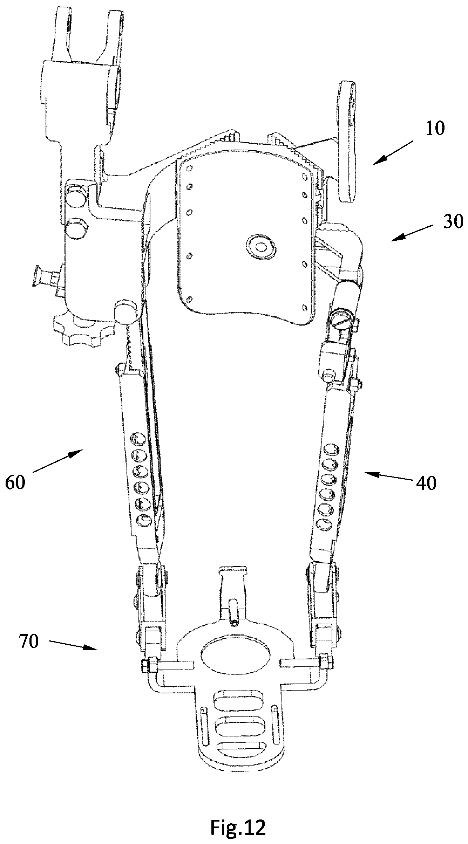

Fig.8 shows the connection of parts 41 and 42 of the first strut in the neutral position. Fig.9 and Fig.12 show the position of the ankle link with the abducted foot support 70. Meanwhile, the position of part 41, element 44 of part 42 of the first strut and the setting screws 48 are shown in Fig. 10 and Fig. 11.

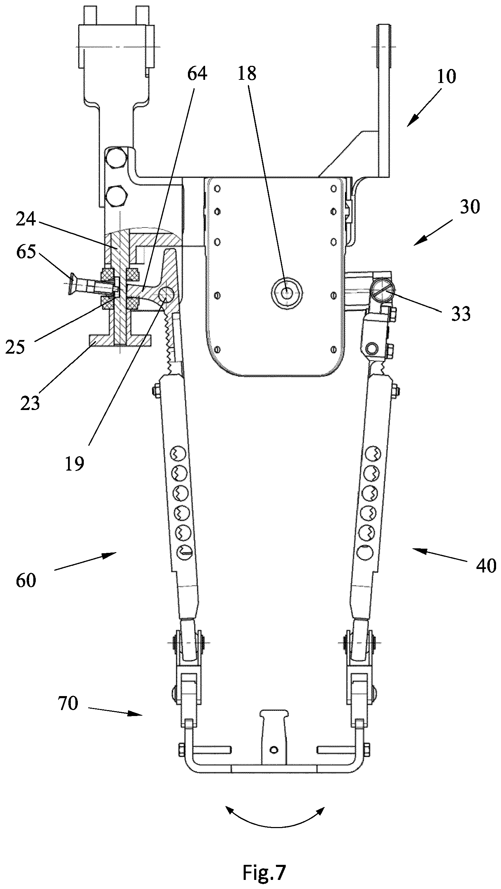

The second strut 60 contains the first element 61, mounted on the pin 19, and the second element 62. At the lower end of the second element 62 there is a spherical hinge in the form of a standard spherical bearing 63. As shown in Fig.7, in this execution example, the strut 60 has a stop 64 made on the first element 61 and intended for initial adjustment of the position of the second strut 60. Adjustment is made by a flywheel 23 with a screw 24 installed in the bracket 12 and passing through the stop 64. The screw 24 has a groove 25 designed to accommodate the spring-loaded shank of the standard spring lock 65, mounted on the stop 64.

Changing the length of the struts 40 and 60 for individual human carrier's fit is similar to the bracket 12, element 14 and lever 30 by moving the first elements 44 and 61 relative to the second elements 45 and 62, respectively. Fixing is performed by teeth made on elements 44, 45 and 61, 62, as well as screws 51 and 66.

The foot support 70 contains a base 71 with brackets 72 and 73 located on the first and second lateral sides of the foot support 70, respectively. The bracket 72 has a lock 75 with a lug 76 designed to accommodate a spherical bearing 49. The bracket 73 has a lock 77 with a lug 78 designed to accommodate a spherical bearing 63.

The connection of the foot support 70 to the first 40 and second 60 struts is made by means of pins 79, 81 located in the holes of the lug 76 and the bearing 49, and the lug 78 and the bearing 63, respectively. Locks 75 and 77 allow quick connection/disconnection of the foot support 70 with struts 40, 60.

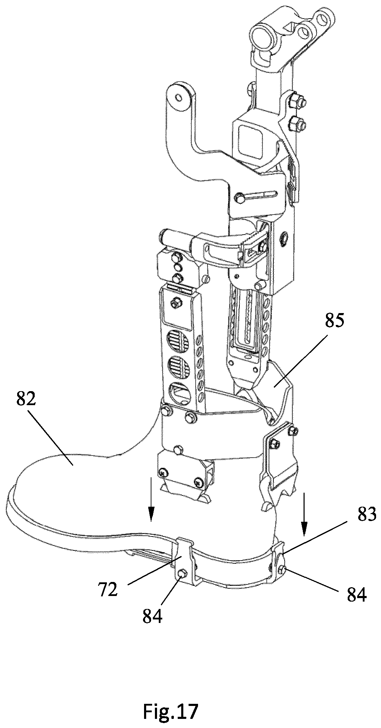

As shown in Fig. 17, to attach the user's shoes 82 to the foot support 70, fasteners in the form of screws 84 are installed on the lateral brackets 72, 73, and on the heel bracket 83. For reliable fixation, counter fasteners, for example, in the form of threaded bushings, can be integrated into the shoes 82.

When a human carrier moves, its ankle joint performs flexion-extension and supination-pronation. Spherical bearings 49 and 63, mounted on the pins 79 and 81, are responsible for the function of flexion-extension in the claimed ankle link.

Six-link mechanism, including the base element 10, the first lever 30, the first strut 40, the second strut 60, and the foot support 70, is responsible for the function of supination-pronation in the claimed ankle link. Such mechanism provides the necessary mobility of the human ankle joint and increased load capacity due to a more uniform distribution of forces on the struts.

It is known from human anatomy that a healthy person can bend the ankle joint (plantar flexion) at an angle of about 50o, straighten the ankle joint (dorsiflexion) at an angle of about 30o. Also, supination at an angle of about 50° and pronation at an angle of about 25°are possible in the ankle joint in a healthy person.

Thus, the angles of rotation of spherical joints and the six-link mechanism should be limited to the above values to prevent injuries when using the ankle link in exoskeletons for patients or in active exoskeletons. Any possible stops, retainers, etc. can be used for restriction. In this case, the stop 36 and screws 22 restrict the supination-pronation, as indicated earlier (Fig.6).

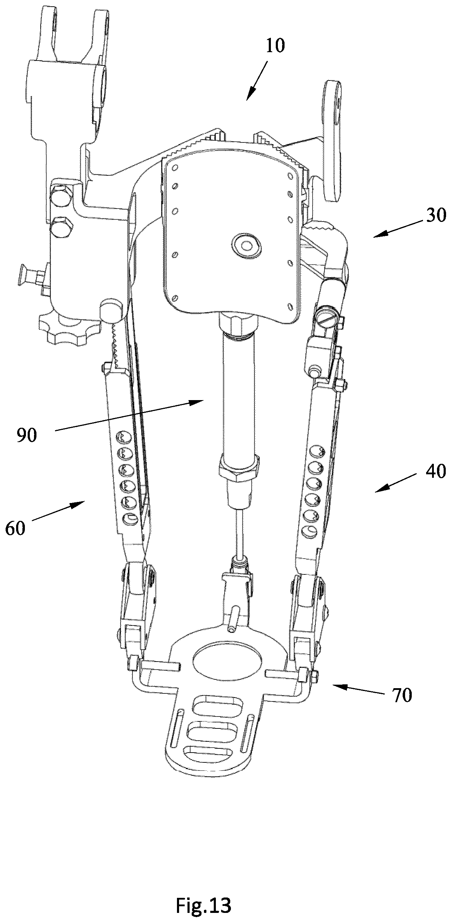

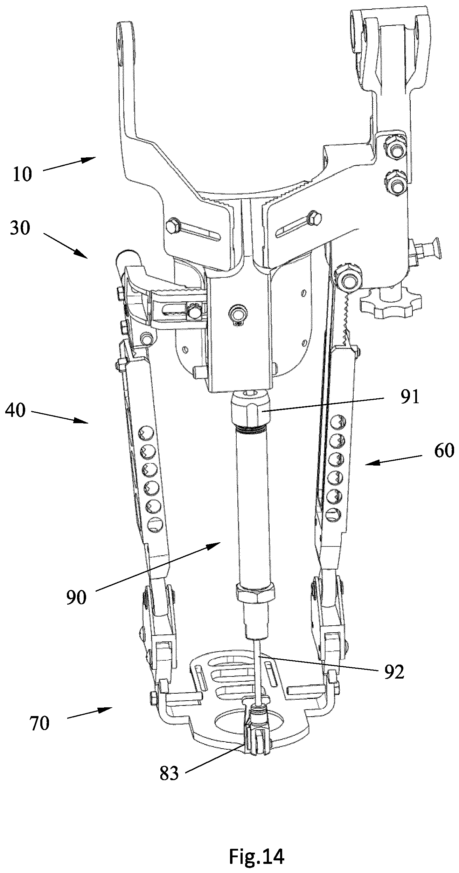

In the described example, the angles of flexion-extension of the ankle link are limited by a pneumatic spring 90, pivotally mounted by the body 91 on the support element 11, and by the rod 92 on the heel bracket 83 of the foot support 70 (Fig. 13, 14). Compression - tension springs or linear displacement drive of various types (electromechanical, hydraulic, pneumatic) can be used instead of the pneumatic spring 90.

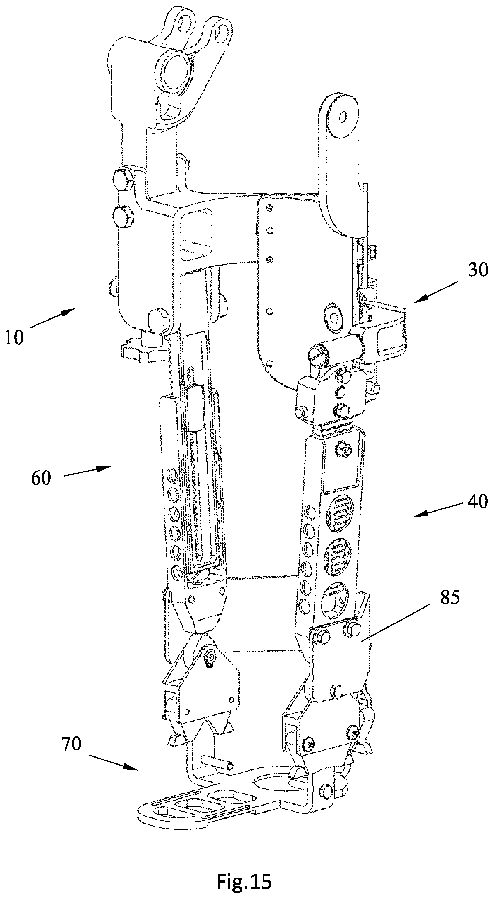

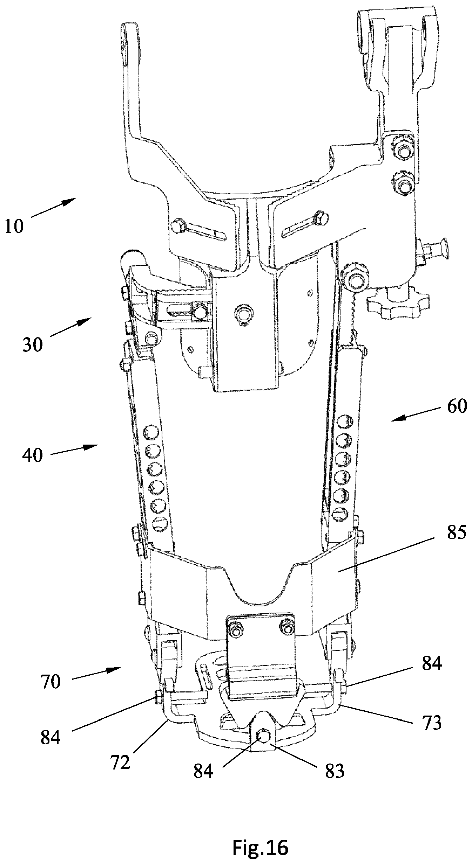

It may be necessary for some patients to completely fix the foot support 70 relative to the struts 40 and 60. As shown in Fig.15 and Fig.16, a bracket 85, rigidly fixed to the struts 40 and 60 and to the heel support bracket 83 of the foot support 70, can be used for this purpose.

Although this document describes various aspects of the implementation of the claimed invention, it is clear to specialists that other approaches to the realization of the presented invention are possible. The different sides and implementation of this invention are presented in this description for illustrative purposes and do not imply limitations, moreover the scope of protection of this invention is specified in the following claims.

## Claims

1. An ankle link for an orthosis or exoskeleton comprising
a base element (10),
a first strut (40),
a second strut (60), the upper end of which is pivotally connected to the base element (10), and
a foot support (70) connected by the first lateral side to the lower end of the first strut (40) and by the second lateral side to the lower end of the second strut (60) by means of hinges having at least two degrees of freedom,
**characterized in that** the ankle link further comprises a first lever (30) mounted on the base element (10) with the possibility of rotation, the upper end of the first strut (40) being pivotally connected to the first lever (30).

2. An ankle link according to claim 1, **characterized in that** the base element (10) is configured with the possibility of connecting to the lower leg of the human carrier.

3. An ankle link according to claim 1, **characterized in that** it is equipped with connecting elements (14, 15) installed on the base element (10), located on the lateral sides of the human carrier's ankle and designed for connection to the knee joint of the ankle link of orthosis or exoskeleton.

4. An ankle link according to claim 3, **characterized in that** the connecting elements (14, 15) are configured with the possibility to adjust their position in the horizontal plane relative to the base element (10).

5. An ankle link according to claim 1, **characterized in that** the first and second struts (40, 60) are configured with the possibility to change their length.

6. An ankle link according to claim 1, **characterized in that** the first lever (30) is configured with the possibility to limit its rotation or to fix in a specified position.

7. An ankle link according to claim 1, **characterized in that** the first lever (30) is configured with the possibility to change its length.

8. An ankle link according to claim 1, **characterized in that** the end of the first lever (30) is rotated and located in a plane parallel to the sagittal plane.

9. An ankle link according to claim 8, **characterized in that** that hinge connection of the first strut (40) to the first lever (30) is formed by the pin (18), located at the end of the first lever (30), and a bearing, located on the first strut (40), and hinge connection of the second strut (40) to the base element (10) is formed by the pin (18), located on the base element (10) in a plane parallel to the sagittal plane, and the bearing located on the second strut (60).

10. An ankle link according to claim 1, **characterized in that** each hinge connecting the corresponding strut (40, 60) to the foot support is configured in the form of a spherical joint.

11. An ankle link according to claim 10, **characterized in that** the first strut (40) is made of two parts mounted with the possibility of rotation relative to each other with subsequent fixation, the first part of the first strut (40) is connected to the first lever (30), and the second part is connected to a spherical joint.

12. An ankle link according to any claim 1 - 11, **characterized in that** it additionally contains a mechanical and/or pneumatic springy element pivotally connected at one end to the base element (10) and at the other end to the heel part of the foot support

13. An ankle link according to any claim 1 - 11, **characterized in that** it additionally contains a linear movement drive pivotally connected at one end to the base element (10), and at the other end to the heel part of the foot support

14. An ankle link according to any claim 1 - 11, **characterized in that** it additionally contains a bracket (83) connected to the heel and lateral sides of the foot support and rigidly fixed to the first and second struts (40, 60).

## Patentansprüche

1. Knöchelverbindung für eine Orthese oder ein Exoskelett, aufweisend ein Basiselement (10),
eine erste Strebe (40),
eine zweite Strebe (60),
dessen oberes Ende schwenkbar mit dem Basiselement (10) verbunden ist,
und
eine Fußstütze (70), welche durch die erste Querseite mit dem unteren Ende der ersten Strebe ( 40) und durch die zweite laterale Seite zum unteren Ende der zweiten Strebe (60) mittels Scharnieren mit mindestens zwei Freiheitsgraden verbunden ist, **dadurch gekennzeichnet,**
**dass** die Knöchelverbindung ferner eine erste Ebene (30) umfasst, die an dem Basiselement (10) drehbar angebracht ist, wobei das obere Ende der ersten Strebe (40) schwenkbar mit der ersten Ebene (30) verbunden ist.

2. Knöchelverbindung nach Anspruch 1,
**dadurch gekennzeichnet,**
**dass** das Basiselement (10) mit Anschlussmöglichkeit an den Unterschenkel des menschlichen Trägers ausgebildet ist.

3. Knöchelverbindung nach Anspruch 1,
**dadurch gekennzeichnet,**
**dass** es mit Verbindungselementen (14, 15) ausgestattet ist, die an dem Basiselement (10) angebracht sind, sich an den lateralen Seiten des Sprunggelenks des menschlichen Trägers befinden und zur Verbindung mit dem Kniegelenk der Knöchelverbindung der Orthese oder des Exoskeletts.

4. Knöchelverbindung nach Anspruch 3,
**dadurch gekennzeichnet,**
**dass** die Verbindungselemente (14, 15) mit der Möglichkeit ausgebildet sind, ihre Position in der horizontalen Ebene relativ zu dem Basiselement (10) einzustellen.

5. Knöchelverbindung nach Anspruch 1,
**dadurch gekennzeichnet,**
**dass** die ersten und zweiten Streben (40, 60) in ihrer Länge veränderbar ausgebildet sind.

6. Knöchelverbindung nach Anspruch 1,
**dadurch gekennzeichnet,**
**dass** der erste Hebel (30) mit der Möglichkeit ausgebildet ist, seine Drehung zu begrenzen oder in einer bestimmten Position zu fixieren.

7. Knöchelverbindung nach Anspruch 1,
**dadurch gekennzeichnet,**
**dass** der erste Hebel (30) in seiner Länge veränderbar ausgebildet ist.

8. Knöchelverbindung nach Anspruch 1,
**dadurch gekennzeichnet,**
**dass** das Ende des ersten Hebels (30) gedreht und in einer Ebene parallel zur Sagittalebene angeordnet ist.

9. Knöchelverbindung nach Anspruch 8,
**dadurch gekennzeichnet,**
**dass** die Gelenkverbindung der ersten Strebe (40) mit dem ersten Hebel (30) durch den am Ende des ersten Hebels (30) befindlichen Stift (18) und einem Lager, das sich an der ersten Strebe (40) befindet, gebildet ist
und die Gelenkverbindung der zweiten Strebe (40) mit dem Basiselement (10) durch den am Basiselement (10) in einer Ebene parallel zur Sagittalebene angeordneten Stift (18) und das daran angeordnete Lager gebildet ist zweite Strebe (60) gebildet ist.

10. Knöchelverbindung nach Anspruch 1,
**dadurch gekennzeichnet,**
**dass** jedes die entsprechende Strebe (40, 60) mit der Fußstütze verbindende Gelenk in Form eines Kugelgelenks ausgebildet ist.

11. Knöchelverbindung nach Anspruch 10,
**dadurch gekennzeichnet,**
**dass** die erste Strebe (40) aus zwei gegeneinander verdrehbar gelagerten Teilen mit anschließender Fixierung besteht,
**dass** der erste Teil der ersten Strebe (40) mit dem ersten Hebel (30) und der zweite Teil ist mit einem Kugelgelenk verbunden ist.

12. Knöchelverbindung nach einem der Ansprüche 1 bis 11,
**dadurch gekennzeichnet,**
**dass** es zusätzlich ein mechanisch und/oder pneumatisch federndes Element enthält, das an einem Ende schwenkbar mit dem Basiselement (10) und am anderen Ende mit dem Fersenteil der Fußstütze verbunden ist.

13. Knöchelverbindung nach einem der Ansprüche 1 bis 11,
**dadurch gekennzeichnet,**
**dass** es zusätzlich einen Linearbewegungsantrieb enthält, der an einem Ende schwenkbar mit dem Basiselement (10) und am anderen Ende mit dem Fersenteil der Fußstütze verbunden ist.

14. Knöchelverbindung nach einem der Ansprüche 1 bis 11,
**dadurch gekennzeichnet,**
**dass** sie zusätzlich eine Klammer (83) enthält, die mit der Ferse und den lateralen Seiten der Fußstütze verbunden und starr an den ersten und zweiten Streben (40, 60) befestigt ist.

## Revendications

1. Lien de cheville pour une orthèse ou un exosquelette comprenant
un élément de base (10),
une première entretoise (40),
une seconde entretoise (60),
dont l'extrémité supérieure est reliée de manière pivotante à l'élément de base (10), et
un support de pied (70)
relié par le premier côté latéral à l'extrémité inférieure de la première jambe de force (40), et
par le second côté latéral à l'extrémité inférieure de la seconde jambe de force (60) au moyen d'articulations ayant au moins deux degrés de liberté,
**caractérisé en ce que** l'articulation de cheville comprend en outre un premier niveau (30) monté sur l'élément de base (10) avec possibilité de rotation, l'extrémité supérieure de la première entretoise (40) étant reliée de manière pivotante au premier niveau (30).

2. Lien de cheville selon la revendication 1, **caractérisé en ce que** l'élément de base (10) est configuré avec la possibilité de se connecter à la jambe inférieure du porteur humain.

3. Lien de cheville selon la revendication 1, **caractérisé en ce qu'**elle est équipée d'éléments de connexion (14, 15) installés sur l'élément de base (10), situés sur les côtés latéraux de la cheville du porteur humain et conçus pour être connectés à l'articulation du genou de la liaison de cheville de l'orthèse ou de l'exosquelette.

4. Lien de cheville selon la revendication 3, **caractérisé en ce que** les éléments de liaison (14, 15) sont configurés avec la possibilité de régler leur position dans le plan horizontal par rapport à l'élément de base (10).

5. Lien de cheville selon la revendication 1, **caractérisé en ce que** la première et la deuxième entretoise (40, 60) sont configurées avec la possibilité de modifier leur longueur.

6. Lien de cheville selon la revendication 1, **caractérisé en ce que** le premier levier (30) est configuré avec la possibilité de limiter sa rotation ou de le fixer dans une position spécifiée.

7. Lien de cheville selon la revendication 1, **caractérisé en ce que** le premier levier (30) est con-figuré avec la possibilité de changer sa longueur.

8. Lien de cheville selon la revendication 1, **caractérisé en ce que** l'extrémité du premier levier (30) est tournée et située dans un plan parallèle au plan sagittal.

9. Lien de cheville selon la revendication 8, **caractérisé en ce que** la connexion articulée de la première jambe (40) au premier levier (30) est formée par l'axe (18), situé à l'extrémité du premier levier (30), et un palier, situé sur la première jambe (40),
et la connexion articulée de la seconde entretoise (40) à l'élément de base (10) est formée par la goupille (18), située sur l'élément de base (10) dans un plan parallèle au plan sagittal, et le palier, situé sur la seconde entretoise (60).

10. Lien de cheville selon la revendication 1, **caractérisé en ce que** chaque charnière reliant l'entretoise correspondante (40, 60) au support de pied est configurée sous la forme d'une articulation sphérique.

11. Lien de cheville selon la revendication 10, **caractérisé en ce que** la première entretoise (40) est constituée de deux parties montées avec la possibilité de rotation l'une par rapport à l'autre avec fixation ultérieure,
la première partie de la première jambe de force (40) est reliée au premier levier (30),
et la seconde partie est reliée à une articulation sphérique.

12. Lien de cheville selon l'une quelconque des revendications 1 à 11, **caractérisé en ce qu'**elle contient en outre un élément élastique mécanique et/ou pneumatique relié de manière pivotante à une extrémité à l'élément de base (10) et à l'autre extrémité à la partie talon du support de pied.

13. Lien de cheville selon l'une quelconque des revendications 1 à 11, **caractérisé en ce qu'**elle contient en outre un entraînement de mouvement linéaire relié de manière pivotante à une extrémité à l'élément de base (10), et à l'autre extrémité à la partie de talon du support de pied.

14. Lien de cheville selon l'une quelconque des revendications 1 à 11, **caractérisé en ce qu'**elle contient en outre un support (83) relié au talon et aux côtés latéraux du support de pied et fixé de manière rigide aux première et seconde entretoises (40, 60).
